# EUROPEAN PATENT APPLICATION

(11) **EP 2 361 648 A1**
(43) Date of publication of application: **31.08.2011**
(21) Application number: 10154006.0
(22) Date of filing: 18.02.2010
(51) Int. Cl.: A61M 5/32, A61M 5/20

(54) **Remover for a protective needle shield**

(71) Applicant: Sanofi-Aventis Deutschland GmbH, 65929 Frankfurt am Main (DE)
(72) Inventor: The designation of the inventor has not yet been filed

(57) **Abstract**

The invention relates to a remover for a protective needle shield (5) for protecting a hollow injection needle (4) of a syringe (3), the syringe (3) arrangeable in an elongate housing (2) of an injection arrangement having an orifice at a proximal end (P) intended to be applied against an injection site, wherein the remover comprises a cap (25) attachable to the proximal end (P) of the housing (2), wherein a resilient clip (26) is attached to the cap (25) for joint axial movement and independent rotation, the resilient clip (26) arranged to extend through the orifice into the housing (2) when the cap (25) is attached to the housing (2), wherein the resilient clip (26) comprises at least two barbs (27) arranged for snapping into a circumferential notch or behind a shoulder of the protective needle shield (5) attached to the hollow needle (4).

## Description

### Technical Field

The invention relates to a remover for a protective needle shield for protecting a hollow injection needle of a syringe.

### Background of the Invention

Administering an injection is a process which presents a number of both mental and physical risks and challenges for users and healthcare professionals.

Injection devices (i.e. devices capable of delivering medicaments from a medication container) typically fall into two categories ― manual devices and auto-injectors.

In a manual device ― the user must provide the mechanical energy to drive the fluid through the needle. This is typically done by some form of button / plunger that has to be continuously pressed by the user during the injection. There are numerous disadvantages to the user from this approach. If the user stops pressing the button / plunger then the injection will also stop. This means that the user can deliver an underdose if the device is not used properly (i.e. the plunger is not fully pressed to its end position). Injection forces may be too high for the user, in particular if the patient is elderly or has dexterity problems.

The extension of the button/plunger may be too great. Thus it can be inconvenient for the user to reach a fully extended button. The combination of injection force and button extension can cause trembling / shaking of the hand which in turn increases discomfort as the inserted needle moves.

Auto-injector devices aim to make self-administration of injected therapies easier for patients. Current therapies delivered by means of self-administered injections include drugs for diabetes (both insulin and newer GLP-1 class drugs), migraine, hormone therapies, anticoagulants etc.

Auto-injectors are devices which completely or partially replace activities involved in parenteral drug delivery from standard syringes. These activities may include removal of a protective syringe cap, insertion of a needle into a patient's skin, injection of the medicament, removal of the needle, shielding of the needle and preventing reuse of the device. This overcomes many of the disadvantages of manual devices. Injection forces / button extension, hand-shaking and the likelihood of delivering an incomplete dose are reduced. Triggering may be performed by numerous means, for example a trigger button or the action of the needle reaching its injection depth. In some devices the energy to deliver the fluid is provided by a spring.

US 2002/0095120 A1 discloses an automatic injection device which automatically injects a pre-measured quantity of fluid medicine when a tension spring is released. The tension spring moves an ampoule and the injection needle from a storage position to a deployed position when it is released. The content of the ampoule is thereafter expelled by the tension spring forcing a piston forward inside the ampoule. After the fluid medicine has been injected, torsion stored in the tension spring is released and the injection needle is automatically retracted back to its original storage position.

Usually the injection needle is equipped with a protective needle shield for keeping the needle sterile and preventing it from being mechanically damaged. The protective needle shield is attached to the needle when the auto-injector or the syringe is assembled. In order to prepare for an injection the user has to remove the protective needle shield and is thus exposed to a high risk of needle stick injuries.

### Summary of the Invention

It is an object of the present invention to provide an improved remover for a protective needle shield for protecting a hollow injection needle of a syringe.

The object is achieved by a remover according to claim 1.

Preferred embodiments of the invention are given in the dependent claims.

According to the invention a remover serves for removing a protective needle shield for protecting a hollow injection needle of a syringe. The syringe is arrangeable in an elongate housing of an injection arrangement having an orifice at a proximal end intended to be applied against an injection site. The remover comprises a cap attachable to the proximal end of the housing. A resilient clip, e.g. consisting of sheet metal is attached to the cap for joint axial movement and independent rotation. The resilient clip is arranged to extend through the orifice into the housing when the cap is attached to the housing. The resilient clip comprises at least two barbs arranged for deflecting during assembly to the housing and for snapping into a circumferential notch or behind a shoulder of the protective needle shield attached to the hollow needle. The remover according to the invention allows for automatically engaging the resilient clip with the protective needle shield during assembly. When the cap is removed from the housing in preparation of an injection the barbs pull out the protective needle shield reliably without exposing the user too high a risk to injure himself. The remover is suited for removing a protective needle shield even if the protective needle shield is arranged far behind the orifice making it impossible to be gripped manually. Thus the needle can be arranged in the housing initially a distance back from the orifice in order to prevent the user from touching the tip of the needle after the protective needle shield is removed.

Preferably the resilient clip comprises a transversal front portion pivoted in the cap, wherein the barbs respectively comprise a longitudinal leg originating from the front portion and ending in an essentially 90° offset hook protruding inward from the longitudinal leg for engaging in the circumferential notch or behind the shoulder. The barbs are inwardly biased in order to reliably grip the protective needle shield.

The front portion may have a central bore for attaching the resilient clip to a pin provided in a front face of the cap. The pin may be deformed to form a mushroom-shaped closing head so as to prevent the resilient clip from being removed from the cap while allowing some clearance for the resilient clip to rotate. Independent rotation of the resilient clip relative to the cap allows for removing the cap by turning it while the resilient clip does not rotate relative to the protective needle shield or needle. Rotation between the protective needle shield and the needle could otherwise result in coring, i.e. material of the protective needle shield being chipped from the protective needle shield, the material migrating into the fluid channel of the hollow needle and obstructing it.

The cap may be attachable to the housing by a screw connection. This allows for a low force removal of the protective needle shield.

The remover may be applied in an auto-injector for administering a dose of a liquid medicament, the auto-injector comprising:
- an elongate housing arranged to contain a syringe with a hollow needle and a stopper for sealing the syringe and displacing the medicament, the housing having a distal end and a proximal end with an orifice intended to be applied against an injection site, wherein the syringe is slidably arranged with respect to the housing,
- driving means capable of, upon activation:
   - pushing the needle from a covered position inside the housing into an advanced position through the orifice and past the proximal end via a plunger,
   - operating the syringe to supply the dose of medicament via the plunger, and
   - retracting the syringe with the needle into the covered position after delivering the medicament.

In the context of this patent application the term proximal refers to the direction pointing towards the patient during an injection while the term distal refers to the opposite direction pointing away from the patient.

The part that travels in the opposite direction in terms of the delay mechanism may be the stopper.

Reliably triggering the retraction of the syringe and needle at the end of an injection normally has to be traded off against an incompletely emptied syringe, which is undesirable. Due to manufacturing tolerances of the syringe and stopper the exact position of the stopper at the end of its travel is not repeatable. Consequently, in some cases the stopper will prematurely bottom out so the retraction will not be triggered at all. In other cases the retraction will be triggered before the stopper bottomed out so residual medicament remains in the syringe.

Releasing the retraction sleeve from the housing a certain amount of time or travel before the stopper bottoms out in the syringe avoids the risk of stalling the retraction by the stopper hitting the end of the syringe prematurely. The damped backward motion of the retraction sleeve due to the delay mechanism allows the plunger and stopper to finish their forward travel so the syringe is entirely emptied. The apertures of the retraction sleeve and the decoupling arms, which are now moving in opposite directions, meet after the stopper and plunger have stopped in order to decouple the decoupling member from the plunger. Due to the gap between the front face and the syringe holder the retraction sleeve is not immediately dragging the syringe back in distal direction when starting to move back. When the retraction sleeve has travelled back far enough to close the gap the stopper has already bottomed out and the plunger has been decoupled from the decoupling member. As soon as the gap is closed the syringe holder, the syringe, the hollow needle and the plunger are dragged back in distal direction.

Thus both problems are solved, reliably retracting the hollow needle to a safe position and fully emptying the syringe which is particularly desirable with expensive drugs. Emptying the syringe is also important for dosage accuracy.

When the delay box is provided with the aforementioned shoulder the retraction motion may be continued undamped after stopper has bottomed out and the decoupling member has been decoupled from the plunger in order to speed up the retraction so the needle rapidly disappears in the housing and risk for needle stick injuries is further reduced.

Preferably the driving means are arranged as a spring means, wherein activating means are arranged to lock the spring means in a pressurized state prior to manual operation and capable of, upon manual operation, releasing the spring means for injection.

The spring means may be a single compression spring arranged to be grounded at a distal end in the housing for advancing the needle and for injecting the dose of medicament. The force of the compression spring is forwarded to the needle and/or the syringe via a plunger. The compression spring is arranged to have its ground in the housing switched to its proximal end for retracting the syringe when the injection of the medicament is at least nearly finished.

The single compression spring is used for inserting the needle, fully emptying the syringe and retracting the syringe and needle to a safe position after injection. Thus a second spring for withdrawing the syringe and needle, which is a motion with an opposite sense compared to advancing the syringe and injecting the dose, is not required. While the distal end of the compression spring is grounded the proximal end moves the syringe forward for inserting the needle and carries on to the injection by pushing on the stopper. When the injection is at least nearly finished the compression spring bottoms out at its proximal end, resulting in the proximal end being grounded in the housing. At the same time the distal end of the compression spring is released from its ground in the housing. The compression spring is now pulling the syringe in the opposite direction.

The auto-injector has a particularly low part count compared to most conventional auto-injectors. The use of just one compression spring reduces the amount of metal needed and consequently reduces weight and costs.

In a preferred embodiment of the invention a retraction sleeve is axially movable arranged in the housing. At least one latch is provided for axially fixing the retraction sleeve in a maximum proximal position. The compression spring is arranged inside the retraction sleeve with its distal end bearing against a distal end face of the retraction sleeve and with its proximal end bearing against a thrust face of a decoupling member. The decoupling member is arranged to decouple the latch when being moved in proximal direction nearly into a maximum proximal position. When decoupled the retraction sleeve is allowed to move in distal direction and retract the needle by means of the spring force which is no longer grounded at its distal end.

Preferably, the plunger is arranged for pushing the syringe and/or the stopper in proximal direction. At least two resilient decoupling arms are arranged at the decoupling member. The decoupling arms exhibit inner ramped surfaces bearing against a first shoulder of the plunger in proximal direction P. The resilient decoupling arms are supportable by an inner wall of the retraction sleeve in order to prevent the decoupling arms from being flexed outward and slip past the first shoulder. In this state the plunger may be pushed in proximal direction by the decoupling member pushing against the first shoulder in order to insert the needle and inject the dose. At least one aperture is arranged in the retraction sleeve allowing the decoupling arms to be flexed outward by the first shoulder thus allowing the first shoulder to slip through the decoupling arms in proximal direction. This may happen when the injection is at least nearly finished. The decoupled plunger allows the syringe and needle to be retracted since it is no longer bearing against the decoupling member.

The syringe may be arranged for joint axial movement with a syringe holder which is slidably arranged in the retraction sleeve. The syringe holder is provided with at least two resilient syringe holder arms arranged distally, the syringe holder arms having a respective inclined surface for bearing against a second shoulder, which is arranged at the plunger proximally from the first shoulder. The syringe holder arms are supportable by an inner surface of the housing in order to prevent them from being flexed outward. Thus, when the trigger button is pressed the spring force forwarded by the plunger does not yet press against the stopper but against the syringe for forwarding it. Consequently, a so called wet injection is avoided, i.e. the liquid medicament is not leaking out of the hollow needle before the needle is inserted. A widened portion is provided in the housing for allowing the syringe holder arms to flex outwards when the syringe holder has nearly reached a maximum proximal position thus allowing the second shoulder to slip through the syringe holder arms and to switch load of the compression spring from the syringe to the stopper. This allows for defining the moment to start injecting the medicament.

A stud may be arranged at the distal end of the plunger. The retraction sleeve may have two or more resilient arms distally from the end face for holding the stud. The stud and/or the resilient arms have ramp features. Thus the resilient arms may be pushed apart by the stud when the plunger is moved in proximal direction. The activating means comprise a trigger button arranged at the distal end of the auto-injector. The trigger button is axially moveable and has at least two rigid retainers for preventing the resilient arms from being flexed outward when the trigger button is in a maximum distal position. Upon pushing the trigger button in proximal direction the retainers are moved in proximal direction in a manner to allow the resilient arms to be flexed out by the stud biased by the compression spring in proximal direction. Thus the stud is allowed to slip past the resilient arms in proximal direction under load of the compression spring in order to start a needle insertion/injection/retraction cycle. The main advantages of this trigger mechanism are its simplicity, the low part count and a high reliability.

In order to reduce the risk of unintentionally triggering the auto-injector a safety button may be arranged laterally at the housing. The safety button has an interlock for preventing the trigger button from being pushed. The safety button is arranged to pull the interlock outward when operated thus allowing the trigger button to be pushed. For this purpose the safety button may be pivoted in the housing or it may be cast in one piece with the housing in a manner to be pivoted somewhere in the middle so pushing one end inwards causes the other end to be pulled outwards.

Consequently, in order to operate the trigger button the safety button has to be pushed first so the auto-injector cannot be operated unintentionally. Another advantage of the lateral safety button is that the risk of operating the auto-injector in the wrong orientation and injecting into the thumb is reduced.

In a preferred embodiment of the invention a delay mechanism is arranged for slowing down the motion of the retraction sleeve. The latches are arranged to be disengaged by the decoupling member before the stopper has reached a maximum proximal position in the syringe. The apertures are arranged to meet the decoupling arms after the stopper has reached its maximum proximal position by means of the motion of the retraction sleeve. A gap is provided between a front face of the retraction sleeve and the syringe holder in their respective maximum proximal positions. The gap allows the retraction sleeve to travel a distance before retracting the syringe holder so the syringe holder is retracted after the decoupling arms met the apertures.

Triggering the retraction when the stopper exactly reaches the end of its travel is a problem due to tolerances when manufacturing the syringe and stopper. Due to these tolerances the position of the stopper at the end of its travel is not repeatable. Consequently, in some cases the stopper would prematurely bottom out so the retraction would not be triggered at all. In other cases the retraction would be triggered before the stopper bottomed so residual medicament would remain in the syringe.

Releasing the retraction sleeve from the housing a certain amount of time or travel before the stopper bottoms out in the syringe avoids the risk of stalling the retraction by the stopper hitting the end of the syringe prematurely. The damped backward motion of the retraction sleeve due to the delay box allows the plunger and stopper to finish their forward travel so the syringe is entirely emptied. The apertures of the retraction sleeve and the decoupling arms, which are now moving in opposite directions, meet after the stopper and plunger have stopped in order to decouple the decoupling member from the plunger. Due to the gap between the front face and the syringe holder the retraction sleeve is not immediately dragging the syringe back in distal direction when starting to move back. When the retraction sleeve has travelled back far enough to close the gap the stopper has already bottomed out and the plunger has been decoupled from the decoupling member. As soon as the gap is closed the syringe holder, the syringe, the hollow needle and the plunger are dragged back in distal direction.

Thus, both problems are solved, reliably retracting the hollow needle to a safe position and fully emptying the syringe which is particularly desirable with expensive drugs. Emptying the syringe is also important for dosage accuracy.

The delay mechanism may comprise a circumferential outer wall with a back collar attached to the housing and a circumferential inner wall with a front collar attached to the retraction sleeve. A cavity is defined between the outer wall and inner wall, the cavity sealed by the back collar and front collar and filled with a viscous fluid. At least one hole is arranged in the delay box for allowing the viscous fluid to be pushed out as the volume of the cavity decreases due to motion of the retraction sleeve. This is a particularly simple and cost-efficient way to damp the backward motion of the retraction sleeve.

In one embodiment of the invention a circumferential shoulder may be arranged between two portions of at least one of the inner wall and the outer wall with the two portions having different cross sections. Thus the cavity is sealed by the respective collar only until the collar reaches the shoulder. When the collar has travelled past the shoulder the cavity is rendered untight so the motion of the retraction sleeve continues undamped from this point, e.g. after the stopper and the plunger have finished their travel in the opposite direction.

The housing may have at least one viewing window for inspecting the syringe.

The auto-injector may preferably be used for subcutaneous or intra-muscular injection, particularly for delivering one of an analgetic, an anticoagulant, insulin, an insulin derivate, heparin, Lovenox, a vaccine, a growth hormone, a peptide hormone, a proteine, antibodies and complex carbohydrates.

The delay mechanism may be employed with other types of auto-injectors. However, the delay mechanism is not restricted to use with auto-injectors. It may be likewise used with other mechanical equipment.

The cap with the sheet metal spring may also be applied with other auto-injectors and injection devices.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will be more fully understood from the detailed description given herein below and the accompanying drawings which are given by way of illustration only, and thus, are not limitive of the present invention, and wherein:
- Figure 1: are two longitudinal sections of an auto-injector with a single compression spring for advancing a syringe with a needle, injecting a dose of medicament and retracting the syringe and needle, the autoinjector as-delivered,
- Figure 2: are two longitudinal sections of the auto-injector with the syringe and needle advanced and the dose expelled from the syringe,
- Figure 3: is a perspective sectional view of the auto-injector in the initial state of figure 1,
- Figure 4: is another perspective sectional view of the auto-injector of figure 3,
- Figure 5: is a detail view of the distal end of the auto-injector with a delay mechanism, and
- Figure 6: is a detailed view of the proximal end of the autoinjector showing the cap and needle shield remover.

Corresponding parts are marked with the same reference symbols in all figures.

### Detailed Description of Preferred Embodiments

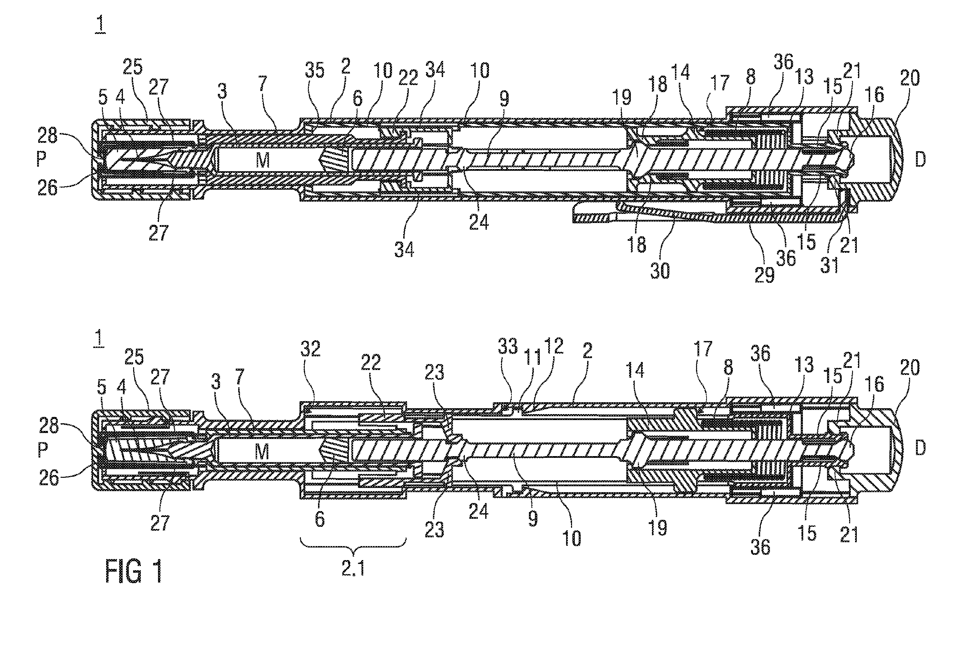

Figure 1 shows two longitudinal sections in different section planes of an auto-injector 1, the different section planes approximately 90° rotated to each other. The auto-injector 1 comprises an elongate housing 2. A syringe 3, e.g. a Hypak syringe, with a hollow needle 4 is arranged in a proximal part of the auto-injector 1. When the auto-injector 1 or the syringe 3 is assembled a protective needle shield 5 is attached to the needle 4. A stopper 6 is arranged for sealing the syringe 3 distally and for displacing a liquid medicament M through the hollow needle 4. The syringe 3 is held in a tubular syringe carrier 7 and supported at its proximal end therein. A single compression spring 8 is arranged in a distal part of the auto-injector 1. A plunger 9 is arranged for forwarding the spring force of the compression spring 8.

Inside the housing 2 a retraction sleeve 10 is slidably arranged. Before the injection is triggered as shown in figure 1 the retraction sleeve 10 is in a maximum proximal position and prevented from moving in distal direction D by means of stops 11 caught behind latches 12 in the housing 2. A distal end of the compression spring 8 bears against an end face 13 of the retraction sleeve 10. Due to the stops 11 and latches 12 the force of the compression spring 8 is thus reacted into the housing 2. The proximal end of the compression spring 8 bears against a decoupling member 14 arranged around the plunger 9. Distally from the end face 13 the retraction sleeve has two or more resilient arms 15 for holding a stud 16 and keeping it from being moved in proximal direction P. The stud 16 is arranged at the distal end of the plunger 9. The stud 16 and the resilient arms 15 have corresponding ramp features for pushing the resilient arms 15 apart in order to allow the stud 16 and the plunger 9 to move in proximal direction P.

The decoupling member 14 comprises a thrust face 17 for bearing against a proximal end of the compression spring 8. Proximally from the thrust face 17 two or more resilient decoupling arms 18 are provided at the decoupling member 14, the decoupling arms 18 having inner ramped surfaces bearing against a first shoulder 19 in the plunger 9 in proximal direction P. The resilient decoupling arms 18 are supported by an inner wall of the retraction sleeve 10 in this situation so they cannot flex outward and slip past the first shoulder 19.

A trigger button 20 is arranged at the distal end D of the auto-injector 1. The trigger button 20 may be pushed in proximal direction P in order to start an injection. As long as the trigger button 20 is not pushed the resilient arms 15 are caught between two or more retainers 21 arranged at the trigger button 20 so the resilient arms 15 cannot flex outward and the stud 16 although proximally biased by the compression spring 8 cannot slip through.

The syringe carrier 7 is engaged for joint axial movement with a syringe holder 22 which is slidably arranged in the retraction sleeve 10. The syringe holder 22 is provided with two or more resilient syringe holder arms 23 arranged distally. The syringe holder arms 23 have a respective inclined surface for bearing against a second shoulder 24 in the plunger 9 arranged proximally from the first shoulder 19. In the initial position shown in figure 1 the syringe holder arms 23 are supported by an inner surface of the housing 2 so they cannot flex outward and the second shoulder 24 cannot slip through. In order to support the syringe holder arms 23 at the housing 2 a respective number of apertures are provided in the retraction sleeve 10.

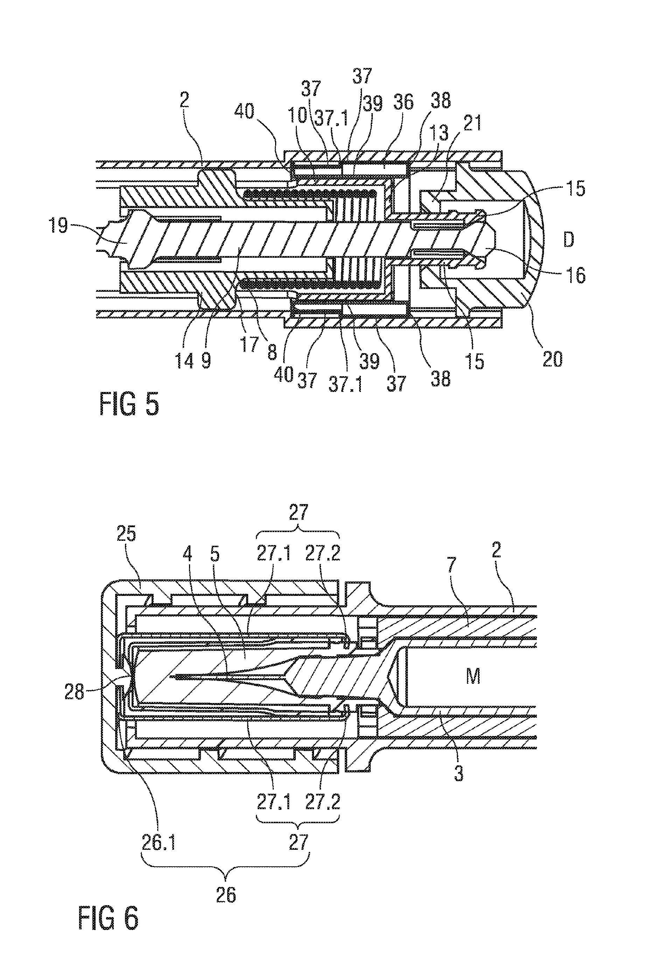

Figure 6 shows details of the proximal end P of the auto-injector 1 as-delivered with a remover for the protective needle shield 5, the remover comprising a cap 25 screwed onto to the proximal end P of the auto-injector 1. The remover further comprises a resilient clip 26 with two or more barbs 27 extending through an orifice into the proximal end P of the auto-injector 1. The resilient clip 26 comprises a transversal front portion 26.1 pivoted in the cap 25. The barbs 27 respectively comprise a longitudinal leg 27.1 originating from the front portion 26.1 and ending in an essentially 90° offset hook 27.2 protruding inward from the longitudinal leg 27.1. The barbs 27 are inwardly biased.

The resilient clip 26 is mounted to the cap 25 for joint axial movement with respect to a longitudinal axis of the auto-injector 1. However, the resilient clip 26 may rotate independently from the cap 25. This may be achieved by attaching the transversal front portion 26.1 with a hole onto a pin protruding inwardly from the cap 25 and deforming the pin to form a mushroom-shaped closing head 28 so as to prevent the resilient clip 26 from being removed while allowing some clearance for the resilient clip 26 to rotate. When the cap 25 is screwed onto the proximal P end of the auto-injector 1 the barbs 27 are pushed down the protective needle shield 5 and snap into a circumferential notch arranged in the protective needle shield 5 or behind a shoulder thereof.

When a user wants to operate the auto-injector 1 the first step is to unscrew the cap 25. Thus the barbs 27 pull the protective needle shield 5 off the syringe 3 in proximal direction P and through the orifice making the syringe 3 ready to be used.

Preferably the resilient clip 26 is a sheet metal clip. Other materials, such as spring wire or plastic may also be applied.

A safety button 29 is arranged laterally at the distal part of the housing 2. The safety button 29 serves for interlocking with the trigger button 20 in a manner to prevent the trigger button 20 from being inadvertently pushed without the safety button 29 being pushed first.

Consequently, in order to operate the trigger button 20 the safety button 29 has to be pushed transversally with respect to the longitudinal axis against the force of a spring element 30 which is formed in the safety button 29. The safety button 29 is pivoted in the middle so pushing the proximal end of the safety button 29 inward pulls an interlock 31 at its proximal end obstructing the trigger button 20 outward so the trigger button 20 can be pushed.

When the trigger button 20 is pushed the retainers 21 are pushed in proximal direction P so the resilient arms 15 are allowed to flex outward. Under load of the compression spring 8 the inclined surfaces of the stud 16 force the resilient arms 15 apart until the stud 16 can slip through.

The second shoulder 24 pushes the syringe holder 22, syringe carrier 7 and syringe 3 forward while no load is exerted onto the stopper 6. The hollow needle 4 appears from the proximal end P and is inserted into an injection site, e.g. a patient's skin.

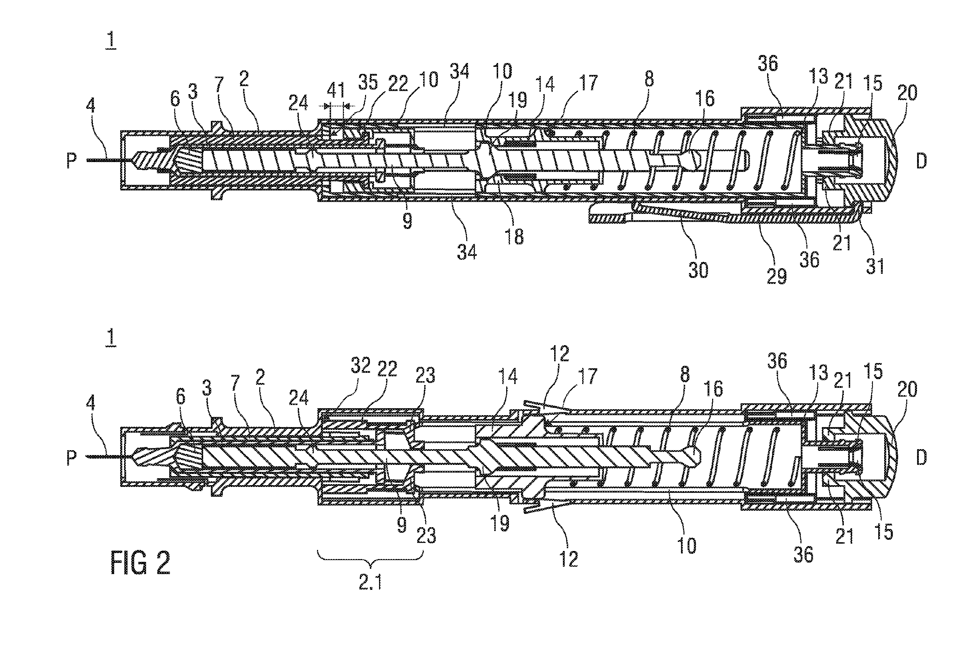

The forward movement continues until the syringe holder 22 bottoms out at a first abutment 32 in the housing 2 (see figure 2). The travel from the initial position (cf. figure 1) up to this point defines an injection depth, i.e. needle insertion depth.

When the syringe holder 22 has nearly bottomed out the resilient syringe holder arms 23 have reached a widened portion 2.1 of the housing 2 where they are no longer supported by the inner wall of the housing 2. However, since the force required to insert the needle 4 is relatively low the second shoulder 24 will continue to drive forward the syringe holder 22 until proximal travel is halted at the first abutment 32. At this point the syringe holder arms 23 are flexed out by the continued force of the second shoulder 24 and allow it to slip through. Now the plunger 9 no longer pushes against the syringe holder 22 but against the stopper 6 for expelling the medicament M from the syringe 3 and injecting it into or through the patient's skin.

When the stopper 6 has nearly bottomed out in the syringe 3 (cf. figure 2) the decoupling member 14 has reached a position where its protrusions push against the latches 12 in a manner to decouple the retraction sleeve 10 from the housing 2, so the retraction sleeve 10 may slide in distal direction D. Thus the compression spring 8 is no longer grounded with its distal end in the housing 2. Instead, as soon as the decoupling member 14 has bottomed out at a second abutment 33 the proximal end of the compression spring 8 gets grounded in the housing while the distal end is pulling the retraction sleeve 10 in distal direction D.

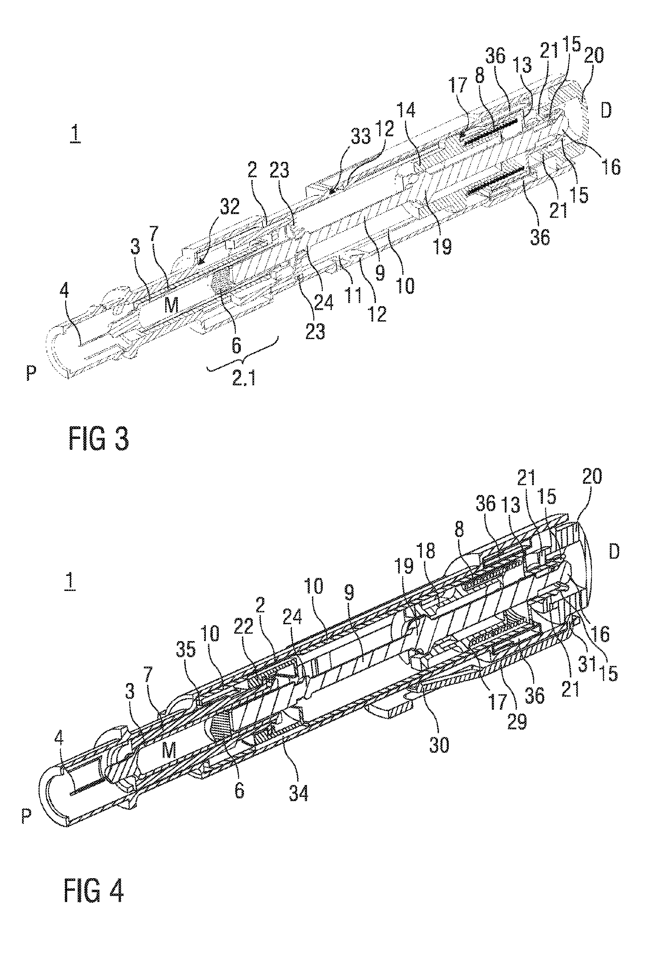

Just before the decoupling member 14 decouples the retraction sleeve 10 from the housing 2 the decoupling arms 18 reach an aperture 34 in the retraction sleeve 10 (see fig. 4) so they are no longer kept from being flexed outward. The decoupling arms 18 are thus pushed outward by the first shoulder 19 pushing against its ramped surfaces so the first shoulder 19 slips through in distal direction as soon as the decoupling member 14 has hit the second abutment 33.

The syringe holder 22 is taken along in distal direction D by the retraction sleeve 10, e.g. by a front face 35. Thus the syringe 3 and needle 4 are retracted into a safe position inside the housing 2, e.g. into the initial position. The plunger 9, no longer bearing against the decoupling arms 18 is pulled back too.

In the distal part of the auto-injector 1 a delay mechanism 36 is arranged (see figure 5 for details). The delay mechanism 36 comprises a circumferential outer wall 37 with a back collar 38 attached to the housing 2 and a circumferential inner wall 39 with a front collar 40 attached to the retraction sleeve 10. A cavity between the outer wall 37 and inner wall 39 is filled with a viscous fluid, such as silicon grease. As the retraction sleeve 10 is moved in distal direction D the inner wall 39 glides along the outer wall 37 wherein the back collar 38 and front collar 40 increasingly reduce the volume of the cavity. One or more holes (not shown) provided in a part of the delay mechanism 36 allow the viscous fluid to be pushed out of the cavity as the volume decreases. The force required to do this slows down the motion of the retraction sleeve 10.

A circumferential shoulder 37.1 is arranged between two portions of the outer wall 37 with the two portions having different cross sections. Thus the cavity is sealed by the front collar 40 only until the front collar 40 reaches the shoulder 37.1. When the front collar 40 has travelled past the shoulder 37.1 the cavity is rendered untight so the motion of the retraction sleeve 10 continues undamped from this point. Preferably this happens after the stopper 6 has bottomed out and after the plunger 9 has been decoupled decoupling member 14.

The retraction sleeve 10 is released by the decoupling member 14 from the housing 2 a certain amount of time or travel before the stopper 6 bottoms out in the syringe 3 so that the apertures 34 of the retraction sleeve 10 and the decoupling arms 18, which are now moving in opposite directions, meet after the stopper 6 and plunger 9 have stopped. The motion of the retraction sleeve 10 is slowed down by the delay mechanism 36. Due to a gap 41 between the front face 35 and the syringe holder 22 the retraction sleeve 10 is not yet dragging the syringe back in distal direction D. The plunger 9 is still pushing against the stopper 6 and expelling residual medicament M. As the stopper 6 hits the proximal end of the syringe 3 the stopper 6 and plunger 9 stop while the retraction sleeve 10 is still slowly moving back in distal direction D. The apertures 34 now meet the decoupling arms 18 allowing them to flex out and the plunger 9 to come clear. The retraction sleeve 10 has now travelled back far enough to close the gap 41 so the syringe holder 22, syringe carrier 7, syringe 3, needle 4 and plunger 9 are dragged back in distal direction D.

The cap 25 and the delay mechanism 36 are not restricted to be used with the auto-injector 1 shown in the embodiments. Instead the cap 25 may be combined with any kind of auto-injector with the needle hidden in the housing prior to an injection. The delay mechanism 36 may be combined with any kind of auto-injector for ensuring full delivery of the syringe's contents and reliable triggering of the retraction, irrespective of the spring means or driving means used in the respective auto-injector.

The housing 2 may have at least one viewing window for inspecting the syringe 3.

The auto-injector 1 may preferably be used for delivering one of an analgetic, an anticoagulant, insulin, an insulin derivate, heparin, Lovenox, a vaccine, a growth hormone, a peptide hormone, a proteine, antibodies and complex carbohydrates.

The delay mechanism 36 is not restricted to use with auto-injectors. It may be likewise used with other mechanical equipment.

The aforementioned arrangement for coupling the plunger (9) to either, the syringe (3) or the stopper (6), may be applied in any auto-injector having a plunger for forwarding a force of a drive means to a syringe with a stopper. The primary advantage of this arrangement ensures the load from the drive means is not transferred directly to the stopper until the needle is inserted in the patient, thus avoiding a wet injection. The arrangement comprises the syringe holder (22) and associated syringe holder arms (23), a shoulder (e.g. the second shoulder 24) on the plunger (9), the support of the holder arms (23) by an inner surface in order to prevent them from flexing out in a first position and, a widened portion (2.1) for allowing them to flex radially and to disconnect from the plunger when in a more proximal position. The spring means or other drive means, the ability to retract the syringe or to forward a needle shroud after injection and other features described herein are not required for the prevention of a wet injection.

### List of References

- 1: auto-injector
- 2: housing
- 2.1: widened portion
- 3: syringe
- 4: hollow needle
- 5: protective needle shield
- 6: stopper
- 7: syringe carrier
- 8: spring means, compression spring
- 8.1: distal end
- 8.2: proximal end
- 9: plunger
- 10: retraction sleeve
- 11: stop
- 12: latch
- 13: end face
- 14: decoupling member
- 15: resilient arm
- 16: stud
- 17: thrust face
- 18: decoupling arm
- 19: first shoulder
- 20: activating means, trigger button
- 21: retainer
- 22: syringe holder
- 23: syringe holder arm
- 24: second shoulder
- 25: cap
- 26: resilient clip
- 26.1: front portion
- 27: barb
- 27.1: longitudinal leg
- 27.2: hook
- 28: closing head
- 29: safety button
- 30: spring element
- 31: interlock
- 32: first abutment
- 33: second abutment
- 34: aperture
- 35: front face
- 36: delay mechanism
- 37: outer wall
- 37.1: shoulder
- 38: back collar
- 39: inner wall
- 40: front collar
- 41: gap
- D: distal end, distal direction
- M: medicament
- P: proximal end, proximal direction

## Claims

1. Remover for a protective needle shield (5) for protecting a hollow injection needle (4) of a syringe (3), the syringe (3) arrangeable in an elongate hou-sing (2) of an injection arrangement having an orifice at a proximal end (P) intended to be applied against an injection site, wherein the remover comprises a cap (25) attachable to the proximal end (P) of the housing (2), wherein a resilient clip (26) is attached to the cap (25) for joint axial movement and independent rotation, the resilient clip (26) arranged to extend through the orifice into the housing (2) when the cap (25) is attached to the housing (2), wherein the resilient clip (26) comprises at least two barbs (27) arranged for snapping into a circumferential notch or behind a shoulder of the protective needle shield (5) attached to the hollow needle (4).

2. Remover according to claim 1, **characterized in that** the resilient clip (26) comprises a transversal front portion (26.1) pivoted in the cap (25), wherein the barbs (27) respectively comprise a longitudinal leg (27.1) originating from the front portion (26.1) and ending in an essentially 90° offset hook (27.2) protruding inward from the longitudinal leg (27.1), wherein the barbs (27) are inwardly biased.

3. Remover according to claim 2, **characterized in that** the front portion (26.1) has a central bore for attaching the resilient clip (26) to a pin provided in a front face of the cap (25).

4. Remover according to claim 3, **characterized in that** the pin is deformed to form a mushroom-shaped closing head (28) so as to prevent the resilient clip (26) from being removed from the cap (25).

5. Remover according to one of the claims 1 to 4, **characterized in that** the cap (25) is attachable to the housing (2) by a screw connection.

6. Auto-injector (1) for administering a dose of a liquid medicament (M), comprising:
- an elongate housing (2) arranged to contain a syringe (3) with a hollow needle (4) and a stopper (6) for sealing the syringe (3) and displacing the medicament (M), the housing (2) having a distal end (D) and a proximal end (P) with an orifice intended to be applied against an injection site, wherein the syringe (3) is slidably arranged with respect to the housing (2),
- driving means (8) capable of, upon activation:
- pushing the needle (4) from a covered position inside the housing (2) into an advanced position through the orifice and past the proximal end (P) via a plunger (9),
- operating the syringe (3) to supply the dose of medicament (M) via the plunger (9), and
- retracting the syringe (3) with the needle (4) into the cover
- a remover according to one of the claims 1 to 5 attachable to the proximal end (P).

7. Auto-injector (1) according to claim 6, **characterized in that** the driving means are arranged as spring means (8), wherein activating means (20) are arranged to lock the spring means (8) in a pressurized state prior to manual operation and capable of, upon manual operation, releasing the spring means (8) for injection.

8. Auto- injector (1) according to claim 7, **characterized in that** the spring means (8) is a single compression spring (8) arranged to be grounded at a distal end (8.1) in the housing (2) for advancing the needle (4) and for injecting the dose of medicament (M) via a plunger (9) and wherein the compression spring (8) is arranged to have its ground in the housing (2) switched to its proximal end (8.2) for retracting the syringe (3).

9. Auto-injector (1) according to claim 8, **characterized in that** a retraction sleeve (10) is axially movable arranged in the housing (2), wherein at least one latch (12) is provided for axially fixing the retraction sleeve (10) in a maximum proximal position, wherein the compression spring (8) is arranged inside the retraction sleeve (10) with its distal end bearing against a distal end face (13) and with its proximal end bearing against a thrust face (17) of a decoupling member (14), wherein the decoupling member (14) is arranged to decouple the latch (12) when being moved in proximal direction (P) nearly into a maximum proximal position, thus allowing the retraction sleeve (10) to move in distal direction (D) and retract the needle (4).

10. Auto-injector (1) according to claim 9, **characterized in that** at least two resilient
decoupling arms (18) are arranged at the decoupling member (14), the decoupling arms (18) having inner ramped surfaces bearing against a first shoulder (19) of the plunger (9) in proximal direction (P), wherein the resilient decoupling arms (18) are supportable by an inner wall of the retraction sleeve (10) in order to prevent the decoupling arms (18) from being flexed outward and slip past the first shoulder (19) and wherein at least one aperture (34) is arranged in the retraction sleeve (10) allowing the decoupling arms (18) to be flexed outward by the first shoulder (19) thus allowing the first shoulder (19) to slip through the decoupling arms (18) in proximal direction (P), wherein the plunger (9) is arranged for pushing the syringe (3) and/or the stopper (6) in proximal direction (P).

11. Auto-injector (1) according to claim 10, **characterized in that** the syringe (4) is
arranged for joint axial movement with a syringe holder (22) which is slidably arranged in the retraction sleeve (10), wherein the syringe holder (22) is provided with at least two resilient syringe holder arms (23) arranged distally, the syringe holder arms (23) having a respective inclined surface for bearing against a second shoulder (24), which is arranged at the plunger (9) proximally from the first shoulder (19) wherein the syringe holder arms (23) are supportable by an inner surface of the housing (2) in order to prevent them from being flexed outward and wherein a widened portion (2.1) is provided in the housing (2) for allowing the syringe holder arms (23) to flex outwards when the syringe holder (22) has nearly reached a maximum proximal position thus allowing the second shoulder (24) to slip through the syringe holder arms (23) and to switch load of the compression spring (8) from the syringe (3) to the stopper (6).

12. Auto-injector (1) according to one of the claims 9 to 11, **characterized in that** a stud (16) is arranged at the distal end of the plunger (9), wherein the retraction sleeve (10) has two or more resilient arms (15) distally from the end face (13) for holding the stud (16), the stud (16) and/or the resilient arms (15) having ramp features so the resilient arms (15) may be pushed apart by the stud (16) when the plunger (9) is moved in proximal direction (P), wherein the activating means comprise a trigger button (20) arranged at the distal end (D) of the auto-injector (1), the trigger button (20) axially moveable and having at least two rigid retainers (21) for preventing the resilient arms (15) from being flexed outward when the trigger button (20) is in a maximum distal position and wherein, upon pushing the trigger button (20) in proximal direction (P) the retainers (21) are moved in proximal direction (P) in a manner to allow the resilient arms (15) to be flexed out by the stud (16) biased by the compression spring (8) in proximal direction (P) thus allowing the stud (16) to slip past the resilient arms (15) in proximal direction (P).

13. Auto-injector (1) according to claim 12, **characterized in that** a safety button (29) is
arranged laterally at the housing (2), the safety button (29) having an interlock (31) for preventing the trigger button (20) from being pushed, the safety button (29) arranged to pull the interlock (31) outward when operated thus allowing the trigger button (20) to be pushed.

14. Auto-injector (1) according to one of the claims 10 to 13, **characterized in that** a
delay mechanism (36) is arranged for slowing down the motion of the retraction sleeve (10), wherein the latches (12) are arranged to be disengaged by the decoupling member (14) before the stopper (6) has reached a maximum proximal position in the syringe (3), wherein the apertures (34) are arranged to meet the decoupling arms (18) after the stopper (6) has reached its maximum proximal position by means of the motion of the retraction sleeve (10) and wherein a gap (41) is provided between a front face (35) of the retraction sleeve (10) and the syringe holder (22) in their respective maximum proximal positions, the gap (41) allowing the retraction sleeve (10) to travel a distance before retracting the syringe holder (22) so the syringe holder (22) is retracted after the decoupling arms (18) met the apertures (34).

15. Auto-injector (1) according to claim 14, **characterized in that** the delay mechanism (36) comprises a circumferential outer wall (37) with a back collar (38) attached to the housing (2) and a circumferential inner wall (39) with a front collar (40) attached to the retraction sleeve (10) wherein a cavity is defined between the outer wall (37) and inner wall (39), the cavity sealed by the back collar (38) and front collar (40) and filled with a viscous fluid and wherein at least one hole is arranged in the delay mechanism (36) for allowing the viscous fluid to be pushed out as the volume of the cavity decreases due to motion of the retraction sleeve (10).
